# FASCICULE DE BREVET EUROPEEN

(11) **EP 1 425 544 B1**
(45) Date de publication et mention de la délivrance du brevet: **20.02.2008**
(21) Numéro de dépôt: 02797994.7
(22) Date de dépôt: 28.08.2002
(51) Int. Cl.: F25J 3/02

(54) **PROCEDE ET INSTALLATION DE FRACTIONNEMENT DE GAZ ISSUS DE LA PYROLYSE D'HYDROCARBURES**
VERFAHREN UND INSTALLATION ZUR FRAKTIONIERUNG EINES DURCH PYROLYSE VON KOHLENWASSERSTOFFEN GEWONNEN GASES
METHOD AND INSTALLATION FOR FRACTIONATING GAS DERIVED FROM PYROLYSIS OF HYDROCARBONS

(30) Priorité: 13.09.2001 FR 0111867
(43) Date de publication de la demande: 09.06.2004
(73) Titulaire: Technip France, 92400 Courbevoie (FR)
(72) Inventeur: PARADOWSKI, Henri, F-95800 Cergy (FR)
(74) Mandataire: Blot, Philippe Robert Emile
(86) Numéro de dépôt international: PCT/FR2002/002952
(87) Numéro de publication internationale: WO 2003/023304

(56) Documents cités:
- US-A- 4 072 485
- US-A- 4 121 917
- US-A- 5 377 490
- DATABASE WPI Section Ch, Week 197948 Derwent Publications Ltd., London, GB; Class H01, AN 1979-86933B XP002196109 & JP 54 136574 A (IDEMITSU PETROCHEM CO), 23 octobre 1979 (1979-10-23)

## Description

La présente invention concerne de façon générale et dans un premier aspect les méthodes de fractionnement de gaz de pyrolyse d' hydrocarbures, et dans un second aspect les installations industrielles et les équipements permettant de mettre en oeuvre ces procédés.

De façon plus précise, la présente invention concerne un procédé de fractionnement d'un gaz issu de la pyrolyse d'hydrocarbures, renfermant de l'hydrogène et des hydrocarbures, en particulier des hydrocarbures de C₁ à C₄ incluant de l'éthylène, de l'éthane et du méthane, éventuellement de l'eau et du CO₂, en au moins un premier courant enrichi en hydrogène et en méthane, éventuellement au moins un second courant composé essentiellement d'eau, et en au moins un troisième courant contenant la plus grande partie des autres hydrocarbures en C₂ et supérieur, en particulier l'éthylène et l' éthane du type décrit dans le préambule de la revendication 1.

Selon un autre aspect, l'invention concerne une installation de fractionnement d'un gaz issu de la pyrolyse d'hydrocarbures, renfermant de l'hydrogène et des hydrocarbures, en particulier des hydrocarbures de C₁ à C₄ incluant de l'éthylène, de l'éthane, et du méthane, éventuellement de l'eau et du CO₂, en au moins un premier courant enrichi en hydrogène et en méthane, éventuellement au moins un second courant composé essentiellement d'eau, et en au moins un troisième courant contenant la plus grande partie des autres hydrocarbures en C₂ et supérieur, en particulier l'éthylène et l'éthane, du type d'écrit dans le préambule de la revendication 3.

Ledit procédé met en jeu, et ladite installation comprend, un échangeur cryogénique d'une puissance thermique de plusieurs dizaines de MW pour les capacités US - A - 4 121 917 décrit un procédé et une installation du type précité. Il s'agit d'un procédé de fractionnement d'un gaz issu de la pyrolyse d'hydrocarbures dans lequel les liquides collectés à l'opération de refroidissement progressif et de liquéfaction partielle du gaz sont envoyés dans une pluralité de colonnes, chaque colonne étant destinée à remplir une fonction précise (déméthaniseur, préfractionnement, séparation, dééthaniseur). Ce procédé connu pose des problèmes d'économie d'énergie et de flexibilité. de fractionnement de gaz généralement considérées. L'alimentation de cet échangeur en fluide de refroidissement, par exemple de l'éthylène, nécessite des cycles secondaire et tertiaire complexes et coûteux. On estime que pour une installation de fractionnement de gaz de 250 tonnes/heure de capacité, le coût total des boucles secondaire et tertiaire est environ de 8 millions d'Euros par an.

On sait également que le gaz naturel est dans certains cas transporté sous forme liquide et stocké sous cette forme à l'arrivée dans des terminaux. Avant d'être injecté dans un réseau de transport et de distribution, une opération de regazéification du gaz naturel liquide (GNL) est nécessaire. Elle est notamment effectuée dans des échangeurs de chaleur refroidis par circulation d'eau fluviale comme décrit dans le document "How Gaz de France optimizes LNG regasification", May 5, 1986 Oil and Gaz Journal, pp 149 - 154. Le GLSL contient un potentiel frigorifique important du fait de sa température très basse, de l'ordre de moins 100°C, qui dans le cas des opérations de regazéification du type citées ci-dessus n'a jamais été en Europe et en Amérique utilisé ni valorisé depuis 50 ans. Dans certains cas, lorsque la température de l'eau de réfrigération est trop basse, le GNL est regazéifié par combustion d'une partie de gaz, ce qui conduit bien entendu à une surconsommation d'énergie, qui ne va pas dans le sens des exigences actuelles de réduction de la production de gaz à effet de serre.

La présente invention vise à remédier à la situation décrite ci-dessus en utilisant les frigories produites pendant l'opération de regazéification du GNL pour refroidir l'échangeur cryogénique, ce qui permet de réduire très fortement le circuit secondaire de l'installation de fractionnement de gaz de pyrolyse et de supprimer le circuit tertiaire. Il en résulte des économies pour l'utilisateur de cette installation et une forte diminution des quantités de fluides réfrigérants nécessaires à l'installation et présentant une consommation énergétique importante

A cet effet, l'invention a pour objet un procédé selon la revendication 1. Le procédé selon l'invention peut comprendre une ou plusieurs des caractéristiques qui font l'objet des revendications 2 à 4.

Dans un second aspect, l'invention a en outre pour objet une installation selon la revendication 5 L'installation selon l'invention peut comprendre l'une ou plusieurs des caractéristiques qui font l'objet des revendications 6 à 8.

D'autres caractéristiques avantageuses de l'invention ressortiront de la description qui en est faite ci-après, à titre indicatif et nullement limitatif, en référence au dessin annexé :
- la planche 1/1 est un schéma de principe d'une installation de fractionnement de gaz de pyrolyse d'hydrocarbures suivant l'invention.

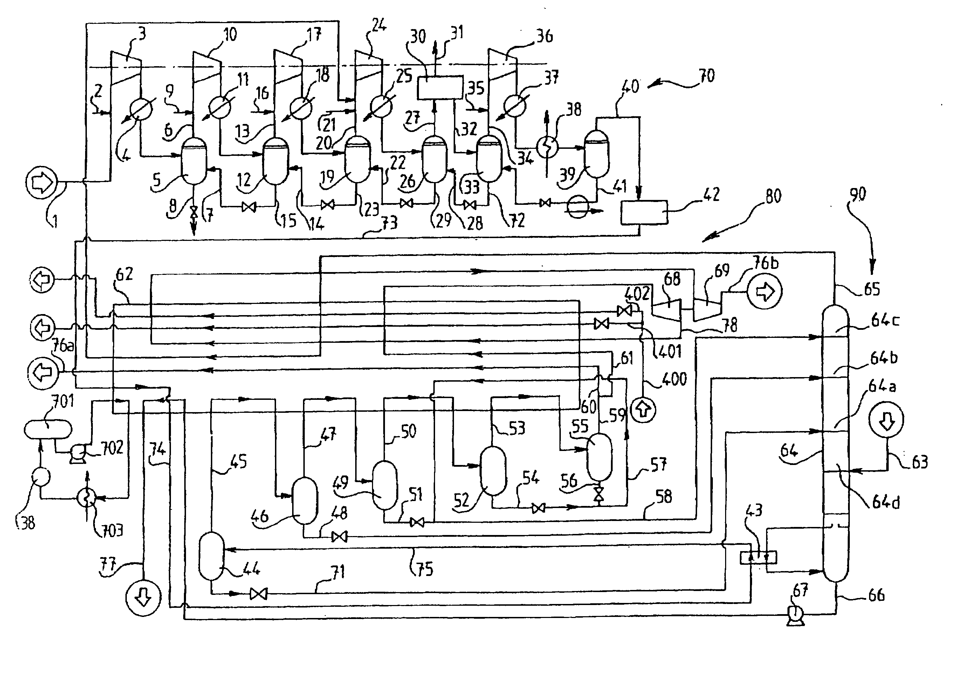

L'alimentation en gaz issus de la pyrolyse d'hydrocarbures est représentée par la ligne 1 de la figure 1. Les caractéristiques de ce gaz sont typiquement les suivantes : température 40°C, pression 1.5 bars absolus, débit massique total environ 257 tonnes/heure, les débits massiques en kg/h des principaux composants de ce flux étant environ :

| Eau | H₂ | CO | CO₂ | Méthane | Ethylène | Ethane |
|---|---|---|---|---|---|---|
| 12200 | 9800 | 260 | 40 | 11700 | 127100 | 83700 |

| Propène | 1,3-Butadiène | n-Butane | Benzène |
|---|---|---|---|
| 2700 | 3100 | 1400 | 3500 |

Les valeurs de débits, de températures et de pressions indiquées dans la description ci-dessous sont des valeurs obtenues par simulation numérique du procédé dans un mode de réalisation typique.

Le flux 1 subit d'abord une opération 70 comprenant 5 étapes de compression et de séchage presque identiques, et une étape d'élimination du CO2.

Au cours de la première étape, le flux 1 reçoit un léger appoint d'eau 2, puis est comprimé par un compresseur 3 à une pression de 3 bars absolus, ce qui entraîne une élévation conjointe de la température à 93°C. Ce flux 1 est ensuite refroidi à 45°C dans un échangeur de chaleur 4 à eau, provoquant la condensation d'une partie de la vapeur d'eau. Le flux 1 alimente alors un séparateur 5 permettant de séparer un flux de tête 6 représentant sensiblement la fraction gazeuse du flux 1, et un flux de pied 8 où se retrouvent les condensats du flux 1. Le séparateur 5 est également alimenté par un flux essentiellement liquide 7, constitué des condensats des étapes suivantes de séchage, qui passent quasi intégralement dans le flux 8.

Le flux 6 subit alors une deuxième étape de traitement comprenant, comme la première étape, un appoint d'eau 9, une compression à 5,3 bars absolus et 91°C par un compresseur 10, un refroidissement à 45°C par un échangeur 11, puis une séparation dans un séparateur 12. Le séparateur 12 est également alimenté par un flux essentiellement liquide 14, constitué des condensats des étapes suivantes de séchage. Ces flux sont séparés en un flux de tête gazeux 13 et en un flux de pied liquide 15, qui, après une détente, constitue le flux 7 d'alimentation du séparateur 5.

Le flux 13 subit une troisième étape de traitement comprenant, comme la première étape, un appoint d'eau 16, une compression à 10,1 bars absolus et 91°C par un compresseur 17, un refroidissement à 45°C par un échangeur 18, puis une séparation dans un séparateur 19. Le séparateur 19 est également alimenté par un flux essentiellement liquide 22, constitué des condensats des étapes suivantes de séchage. Ces flux sont séparés en un flux de tête gazeux 20 et en un flux de pied liquide 23, qui, après une détente, constitue le flux 14 d'alimentation du séparateur 12.

Le flux gazeux 20 reçoit un appoint d'eau 21 et est mélangé à un flux gazeux recyclé 65 venant de la tête d'une colonne de distillation 64. Ce flux 65 sera décrit plus bas. Son débit massique est d'environ 27900 kg/h, sa pression de 10 bars absolus et sa température de 40°C. Ces trois flux combinés subissent comme précédemment une compression à 19,3 bars absolus et 90°C par un compresseur 24, un refroidissement à 45°C par un échangeur 25, puis une séparation dans un séparateur 26. Le séparateur 26 est également alimenté par un flux essentiellement liquide 28, constitué des condensats des étapes suivantes de séchage. Ces flux sont séparés en un flux de tête gazeux 27 et en un flux de pied liquide 29, qui, après une détente, constitue le flux 22 d'alimentation du séparateur 19.

Le flux gazeux 27 subit alors une opération d'élimination du CO2 par lavage par de la soude dans l'équipement 30, générant un flux 31 comprenant au moins la quasi-totalité du CO2 initialement contenu dans le flux 27, et un flux 32 appauvri en CO2 qui alimente ensuite un séparateur 33. Le séparateur 33 est également alimenté par un flux essentiellement liquide 41, constitué des condensats de la dernière étape de séchage. Ces flux sont séparés en un flux de tête gazeux 34 et un flux de pied liquide 72, qui, après une détente, constitue le flux 28 d'alimentation du séparateur 26.

Le flux gazeux 34 reçoit un appoint d'eau 35 et subit comme précédemment une compression à 34,3 bars absolus et 88°C par un compresseur 36, un refroidissement à 45°C par un échangeur 37, puis un second refroidissement à 15°C par un échangeur 38 refroidi par un cycle butane qui sera décrit plus bas, et enfin une séparation dans un séparateur 39. Le flux 34 est séparé en un flux de tête gazeux 40 et en un flux de pied liquide 41, qui, après refroidissement et détente, alimente le séparateur 33.

Le flux gazeux 40 subit alors une dernière déshydratation dans le dessicateur 42 permettant d'éliminer les traces d'eau restante et donne le flux 73.

L'ensemble des condensats collectés dans les séparateurs 5, 12, 19, 26, 33 et 39 constitue donc le flux 8, qui est aussi le second courant mentionné dans le préambule de la présente demande. Ce flux, d'un débit d'environ 21100 kg/h contient seulement de l'eau et quelques traces d'hydrocarbures.

Les caractéristiques du flux 73 à la fin de l'opération de compression et de séchage 70 sont les suivantes : pression environ 33 bars absolus, température environ 15°C, débit environ 272600 kg/h. Il subit alors une opération 80 de refroidissement progressif et de liquéfaction partielle comprenant cinq étapes presque identiques de refroidissement dans un échangeur cryogénique 62 et de séparation gaz/liquide.

Le flux 73 est refroidi et partiellement liquéfié une première fois dans l'échangeur cryogénique 62. Dans ce cas, chaque hydrocarbure est liquéfié en proportion croissante avec son poids moléculaire, le méthane étant liquéfié en très faible proportion, et l'hydrogène ne l'étant quasiment pas. Le flux résultant 74 passe ensuite dans un échangeur de chaleur 43 à contre-courant, qui amène sa température à moins 36°C et contribue à liquéfier plus complètement le gaz, le fluide réfrigérant étant le liquide de pied 66 de la colonne à distiller 64 qui sera décrite plus bas.

Le flux refroidi 75 obtenu alimente ensuite un séparateur gaz/liquide 44 séparant le flux 40 en un flux gazeux 45 et un flux liquide 71. Le flux liquide 71 subit une détente à 11,5 bars absolus ce qui amène sa température à environ moins 48°C et alimente un étage central 64a de la colonne à distiller 64. Il est composé essentiellement d'éthylène et d'éthane et contient une forte majorité des hydrocarbures en C₃ et plus amenés par le flux 1.

Le flux gazeux 45 est refroidi et partiellement liquéfié une seconde fois dans l'échangeur cryogénique 62. La quantité résiduelle d'hydrocarbures en C₃ et plus entraînée avec le flux gazeux 45 est liquéfiée dans sa quasi-totalité. Une forte proportion de l'éthane et. de l'éthylène contenus dans le flux 45 est liquéfiée, ainsi qu'une fraction du méthane comprise entre 10 et 15 %, l'hydrogène ne l'étant quasiment pas.

Le flux 45 alimente ensuite un séparateur gaz/liquide 46 séparant le flux 45 en un flux gazeux 47 et un flux liquide 48. Le flux liquide 48 subit une détente à 11,5 bars absolus ce qui amène sa température à environ moins 71°C et alimente un deuxième étage 64b de la colonne à distiller 64 situé au dessus de l'étage 64a d'alimentation par le flux 71. Il est composé essentiellement d'éthylène et d'éthane et contient du méthane et la quasi-totalité des hydrocarbures en C₃ et plus non liquéfiés à la première étape de refroidissement.

Le flux gazeux 47 est refroidi et partiellement liquéfié une troisième fois dans l'échangeur cryogénique 62 à environ moins 95°C. Une forte proportion de l'éthane et de l'éthylène entraînés dans le flux 47 est liquéfiée, ainsi qu'une fraction du méthane comprise entre 10 et 20 %, l'hydrogène ne l'étant quasiment pas.

Le flux 47 alimente ensuite un séparateur gaz/liquide 49 séparant le flux 47 en un flux gazeux 50 et un flux liquide 51. Le flux liquide 51 subit une détente à 11,5 bars absolus ce qui amène sa température à environ moins 95°C et, après mélange avec des flux liquéfiés aux deux étapes suivantes de refroidissement, alimente un étage de tête 64c de la colonne à distiller 64. Ce flux 51 est composé essentiellement d'éthylène et d'éthane et contient du méthane.

Le flux gazeux 50 est refroidi et partiellement liquéfié une quatrième fois dans l'échangeur cryogénique 62. ce qui amène sa température à environ moins 115°C. Une forte proportion de l'éthane et de l'éthylène entraînés dans le flux 50 est liquéfiée, ainsi fraction du méthane inférieure à 15%, l'hydrogène ne l'étant quasiment pas.

Le flux 50 alimente ensuite un séparateur gaz/liquide 52 séparant le flux 50 en un flux gazeux 53 et un flux liquide 54. Le flux liquide 54 subit une détente à 11,5 bars absolus ce qui amène sa température à environ moins 121°C et se mélange au flux 56 liquéfié à la dernière étape de refroidissement. Ce flux 54 est composé essentiellement d'éthylène et d'éthane et contient du méthane.

Le flux gazeux 53 est refroidi et partiellement liquéfié une cinquième fois dans l'échangeur cryogénique 62 à environ moins 135°C. La quasi-totalité de l'éthane et de l'éthylène entraînés dans le flux 53 est liquéfiée, ainsi qu'une fraction du méthane inférieure à 15%, l'hydrogène ne l'étant quasiment pas.

Le flux 53 alimente ensuite un séparateur gaz/liquide 55 séparant le flux 53 en un flux gazeux 59 et un flux liquide 56. Le flux liquide 56 subit une détente à 11,5 bars absolus et se mélange au flux 54 pour former le flux 57. Le flux 56 est composé essentiellement d'éthylène et d'éthane et contient du méthane. Le flux 57 est réchauffé à moins 98°C par passage dans l'échangeur cryogénique 62, mélangé au flux 51, et alimente un étage de tête 64c de la colonne à distiller 64.

Après cette cinquième étape de refroidissement, le flux 59 ne contient plus en quantité notable que de l'hydrogène et du méthane, les autres hydrocarbures étant à l'état de traces. En particulier l'éthane et l'éthylène ont été liquéfiés à plus de 99%. Son débit massique est d'environ 22000 kg/h dont 9900 kg/h d'hydrogène et 11400 kg/h de méthane. Sa pression est de 31,3 bars absolus environ et sa température de moins 135°C environ.

Ce flux 59 est divisé en deux flux 60 et 61, le flux 60 ne représentant que quelques % du débit massique total. Le flux 60 se réchauffe dans l'échangeur 62 à une température de 40°C puis est collecté, ce flux étant une partie 76a du premier courant 76 mentionné dans l'introduction. Le flux 61 passe également dans l'échangeur 62, se réchauffe à moins 104°C et se détend dans une turbine 68 à une pression de 7,7 bar et moins 150°C environ. Le flux détendu 78 se réchauffe ensuite une deuxième fois dans l'échangeur 62 à 0°C et est comprimé à 10,8 bars absolus et 41°C environ par un compresseur 69 couplé à la turbine 68. Il est ensuite collecté et constitue une autre partie 76b du premier courant 76.

Les flux de gaz 71, 48, et 58 liquéfiés pendant l'opération 80 de liquéfaction et de refroidissement subissent une opération 90 de distillation permettant essentiellement de séparer le méthane de l'éthylène et de l'éthane.

Comme indiqués ci-dessus ces flux sont injectés dans la colonne à distiller 62 à trois étages différents 64a, 64b et 64c. Un flux 63 d'un débit massique de 1000 kg/h environ contenant essentiellement de l'éthylène est injecté à un étage 64d situé en dessous de l'étage 64a où est introduit le flux 71. Ce flux 63 provient de la compression des gaz d'évaporation d'un stockage cryogénique et atmosphérique d'éthylène liquide obtenu par une séparation plus poussée du flux résultant de l'opération 90, cette étape de procédé n'étant pas couverte par le présent brevet et n'étant en conséquence pas décrite. Cette colonne permet de séparer un flux de tête 65 ayant un débit massique d'environ 27900 kg/h, une température de moins 71°C environ sous une pression de 11,3 bars absolus, et un flux de pied 66 ayant un débit massique d'environ 223700 kg/h, une température de moins 41°C environ sous une pression de 11,3 bars absolus. Le flux gazeux 65 contient la quasi-totalité du méthane et de l'hydrogène et une faible fraction de l'éthylène et de l'éthane venant des flux 71,48 et 58, et quasiment pas d'hydrocarbures en C3 et plus. Il est recyclé au quatrième étage de compression de l'opération 70. Le flux liquide 66 contient la plus grande partie de l'éthylène, de l'éthane et la quasi-totalité des hydrocarbures en C3 et plus venant des flux 71,48, et 58, et ne contient quasiment pas de méthane et d'hydrogène. Ce flux 66 est comprimé par la pompe 67 à 25 bars, réchauffé dans l'échangeur 62 à moins 11°C environ, puis collecté. Les débits massiques des principaux constituants sont les suivants.

| Eau | H₂ | CO | CO₂ | Méthane | Ethylène | Ethane |
|---|---|---|---|---|---|---|
| 0 | 0 | 0 | 0 | 9 | 127100 | 83700 |

| Propène | 1,3 Butadiène | n-Butane | Benzène |
|---|---|---|---|
| 2700 | 3100 | 1400 | 3500 |

Il constitue le troisième courant 77 défini dans le préambule.

Le refroidissement de l'échangeur principal 62 est principalement assuré par un flux d'alimentation 400 de GNL sous environ 52 bar relatif. La température d'alimentation du GNL peut varier typiquement entre moins 89 et moins 160°C, le débit nécessaire pour apporter le pouvoir réfrigérant variant en conséquence entre 100800 et 176600 kg/h.

Le flux 400 est ensuite séparé en un flux moyenne pression 401 et un flux haute pression 402, le flux 401 subissant une détente à 30 bars qui abaisse sa température. Le débit du flux 401 dépend de la température d'alimentation du GNL, ce débit étant d'autant plus grand que la température est faible .

Dans un mode de réalisation où la température d'alimentation est de moins 89°C, le débit du flux 401 est de 17700 kg/h environ, et la température du GNL après détente à 30 bars absolus est de moins 99°C. Dans ce cas, la réfrigération nécessaire au refroidissement du flux 50 à moins 115°C et du flux 53 à moins 135°C viennent du flux 61 après détente dans la turbine 68.

Dans un mode de réalisation où la température d'alimentation est de moins 160°C, le débit du flux 401 est nul.

Les flux 402 et/ou 401 alimentent l'échangeur cryogénique 62, et subissent une évaporation complète. La réfrigération cédée pendant cette évaporation sert au refroidissement de différents flux traversant l'échangeur 62. La température de sortie des flux 401 et 402 est environ de 30°C.

Un cycle de récupération de froid annexe typiquement au butane comprenant au moins une cuve de stockage 701 et une pompe de circulation 702 assure le refroidissement de l'échangeur 38 et éventuellement d'autres échangeurs 703. L'échangeur cryogénique 62 joue le rôle d'échangeur apportant du froid à ce cycle.

Dans les opérations 70, 80 et 90 décrites ci-dessus, le CO contenu dans le flux 1 se comporte sensiblement comme l'hydrogène, et les éventuels hydrocarbures en C2 se comportent sensiblement comme l'éthylène et l'éthane.

Dans une variante de réalisation, les compresseurs 3, 10, 17, 24 et 36 peuvent être remplacés par un compresseur multi-étages unique.

Il ressort clairement de cette description que les débits massiques importants de gaz à traiter et les très basses températures nécessaires pour la liquéfaction des gaz de pyrolyse d'hydrocarbures demandent une capacité de refroidissement importante. Les cycles classiques de refroidissement de cette taille sont très coûteux. L'utilisation des frigories cédés par le GNL pendant sa regazéification permet d'éliminer presque entièrement ces cycles. Elle permet de valoriser le pouvoir frigorifique du GNL, qui serait perdu sinon. Les puissances thermiques mises en jeu dans l'art antérieur et selon l'invention 5 sont indiquées approximativement dans le tableau ci-dessous :

| **Cas** | | **art** | **avec GNL selon l'invention** | | |
|---|---|---|---|---|---|
| | | **antérieur** | **cas 1** | **cas 2** | **cas 3** |
| **Réfrigération nécessaire** | | | | | |
| Puissance du cycle | kW | 10800 | 0 | 0 | 0 |
| secondaire | | | | | |
| Puissance du cycle | kW | 9900 | 0 | 0 | 0 |
| tertiaire | | | | | |
| Total | kW | 20700 | 0 | | 0 |
| **Gaz naturel liquéfié** | | | | | |
| utilisé | | | | | |
| Débit de GNL vaporisé | kg/h | 0 | 176600 | 119600 | 100800 |
| Chaleur de vaporisation du | kW | 0 | 27100 | 25800 | 24100 |
| GNL | | | | | |
| Température du GNL | °C | | moins 89.4 | moins 135 | moins 158.8 |
| Pression du GNL | bars | | 52 | 52 | 52 |
| **Gaz naturel produit** | | 0 | | | |
| Température | °C | | 30 | 30 | 30 |
| Gaz à 30 bars | kg/h | | 17600 | 0 | 0 |
| Gaz à 50 bars | kg/h | | 159000 | 119600 | 100800 |

## Revendications

1. Procédé de fractionnement d'un gaz (1) issu de la pyrolyse d'hydrocarbures, renfermant de l'hydrogène et des hydrocarbures, en particulier des hydrocarbures de C₁ à C₄ incluant de l'éthylène, de l'éthane et du méthane, éventuellement de l'eau et du CO₂, en au moins un premier courant (76) enrichi en hydrogène et en méthane, éventuellement au moins un second courant (31) composé essentiellement d'eau, comprenant:
a) une opération (70) de compression et de séchage des gaz (1) par passage dans une série de compresseurs (3, 10, 17, 24, 36) associés chacun à un refroidisseur respectivement (4, 11, 18, 25, 37) et un séparateur respectivement (5, 12, 19, 26, 39), et comprenant en outre une étape d'élimination du CO₂, si le gaz (1) en contient trop, pour l'obtention d'un gaz comprimé (73),
b) une opération (80) de refroidissement progressif et de liquéfaction partielle d'un gaz (73) obtenu à partir de l'opération a) de compression et de séchage, par passages successifs dans un échangeur cryogénique (62) et séparation dans des séparateurs (44, 46, 49,52, 55) en :
(i) des liquides respectivement (71, 48, 51, 54, 56) enrichis en hydrocarbures en C₂ et supérieurs, en particulier en éthylène et en éthane, mais pauvres en hydrogène et en méthane, et en :
(ii) des gaz respectivement (45, 47, 50, 53, 59) appauvris en hydrocarbures en C₂ et supérieurs, en particulier en éthylène et en éthane, chaque passage successif dans l'échangeur cryogénique (62) amenant les gaz (73, 45, 47, 50, 53) à une température relativement plus basse et permettant donc de liquéfier plus complètement les hydrocarbures, le gaz (59) issu du dernier séparateur (55) constituant le premier courant (76), contenant essentiellement de l'hydrogène et du méthane et une très faible quantité d'éthylène et d'éthane, ce gaz (59) étant ensuite collecté, l'échangeur cryogénique (62) étant refroidi par au moins un fluide réfrigérant (400) qui est du gaz naturel liquide (GNL) ; et
c) une opération de distillation (90),
**caractérisé en ce que** :
d) le gaz (73) soumis à l'opération b) de refroidissement progressif et de liquéfaction partielle est le gaz issu de l'opération a) de compression et de séchage ;
e) l'opération de distillation c) est effectuée dans une colonne (64) alimentée à différents niveaux par les liquides (71, 48, 51, 54, 56) collectés à l'opération b) et partiellement détendus, ladite colonne (64) permettant de séparer, dans une partie supérieure relativement plus froide, un courant de tête gazeux (65) enrichi en méthane et appauvri en éthylène et en éthane, et dans une partie inférieure relativement moins froide un courant de pied liquide (66) appauvri en méthane et enrichi en éthylène et en éthane, le courant de tête (65) étant recyclé à l'opération a), en amont du compresseur dont la pression d'entrée est la plus proche de la pression de fonctionnement de la colonne (64), le courant de pied (66) étant collecté et constituant un troisième courant (77), contenant la plus grande partie des hydrocarbures en C₂ et supérieur, en particulier l'éthylène et l'éthane ;
f) la température du GNL (400) à l'entrée de l'échangeur cryogénique (62) peut varier de moins 89°C à moins 160°C ; et
g) le gaz (59) issu du dernier séparateur (55), le courant de tête (65) et le courant de pied (66) sont réchauffés dans l'échangeur cryogénique (62).

2. Procédé de fractionnement d'un gaz issu de la pyrolyse d'hydrocarbures selon la revendication 1, **caractérisé en ce que** le gaz (59) issu du dernier séparateur (55) est divisé en un flux (60) réchauffé dans l'échangeur cryogénique (62) et un flux (61) détendu et refroidi dans une turbine (68) puis réchauffé dans l'échangeur cryogénique (62).

3. Procédé de fractionnement d'un gaz issu de la pyrolyse d'hydrocarbures selon la revendication 1 ou 2, **caractérisé en ce que** l'échangeur cryogénique (62) est alimenté par deux flux de GNL, un flux haute pression (402) à une première pression relativement plus haute et un flux moyenne pression (401) à une seconde pression relativement plus faible.

4. Procédé de fractionnement d'un gaz issu de la pyrolyse d'hydrocarbures selon la revendication 3, **caractérisé en ce que** le flux moyenne pression (401) de GNL alimentant l'échangeur cryogénique (62) est produit en dérivant une partie du flux haute pression (402) et en le détendant pour abaisser sa température, le débit du flux moyenne pression (401) dépendant de la température du flux de GNL (400) et étant d'autant plus grand que cette température est haute.

5. Installation de fractionnement d'un gaz (1) issu de la pyrolyse d'hydrocarbures, renfermant de l'hydrogène et des hydrocarbures, en particulier des hydrocarbures de C₁ à C₄ incluant de l'éthylène, de l'éthane et du méthane, éventuellement de l'eau et du CO₂, en au moins un premier courant (76) enrichi en hydrogène et en méthane, éventuellement au moins un second courant (31) composé essentiellement d'eau, comprenant :
a) une unité (70) de compression et de séchage du gaz (1) par passage dans une série de compresseurs (3, 10, 17, 24, 36) associés chacun à un refroidisseur respectivement (4, 11, 18, 25, 37) et un séparateur respectivement (5, 12, 19, 26, 39), comprenant en outre une étape d'élimination du CO₂ si le gaz (1) en contient trop, pour l'obtention d'un gaz comprimé (73),
b) une unité (80) de refroidissement progressif et de liquéfaction partielle d'un gaz (73) obtenu à partir de l'unité (70) de compression et de séchage, par passages successifs dans un échangeur cryogénique (62) et séparation dans des séparateurs (44, 46, 49, 52, 55) en :
(i) des liquides respectivement (71, 48, 51, 54, 56) enrichis en hydrocarbures en C₂ et supérieurs, en particulier en éthylène et en éthane, mais pauvres en hydrogène et en méthane, et en :
(ii) des gaz respectivement (45, 47, 50, 53, 59) appauvris en hydrocarbures en C₂ et supérieurs, en particulier en éthylène et en éthane, chaque passage successif dans l'échangeur cryogénique (62) amenant les gaz (73, 45, 47, 50, 53) à une température relativement plus basse et permettant donc de liquéfier plus complètement les hydrocarbures, le gaz (59) issu du dernier séparateur (55) constituant le premier courant (76), contenant essentiellement de l'hydrogène et du méthane et une très faible quantité d'éthylène et d'éthane, ce gaz (59) étant ensuite collecté, l'échangeur cryogénique (62) étant refroidi par au moins un fluide réfrigérant (400) qui est du gaz naturel liquide (GNL) ; et
c) une unité de distillation (90) ;
**caractérisée en ce que** :
d) le gaz (73) introduit dans l'unité (80) de refroidissement progressif et de liquéfaction partielle est le gaz issu de l'unité (70) de compression et de séchage ;
e) ladite unité de distillation (90) comprend essentiellement une colonne (64), alimentée à différents niveaux par les liquides (71, 48, 51, 54, 56) collectés dans l'unité (80) de refroidissement progressif et de liquéfaction partielle et partiellement détendus, ladite colonne (64) permettant de séparer, dans une partie supérieure relativement plus froide, un courant de tête gazeux (65) enrichi en méthane et appauvri en éthylène et en éthane, et dans une partie inférieure relativement moins froide un courant de pied liquide (66) appauvri en méthane et enrichi en éthylène et en éthane, le courant de tête (65) étant recyclé dans l'unité (70) de compression et de séchage, en amont du compresseur dont la pression d'entrée est la plus proche de la pression de fonctionnement de la colonne (64), le courant de pied (66) étant collecté et constituant un troisième courant (77), contenant la plus grande partie des hydrocarbures en C₂ et supérieur, en particulier l'éthylène et l'éthane ;
f) la température du GNL (400) à l'entrée de l'échangeur cryogénique (62) peut varier de moins 89°C à moins 160°C ; et
g) le gaz (59) issu du dernier séparateur (55), le courant de tête (65) et le courant de pied (66) sont réchauffés dans l'échangeur cryogénique (62).

6. Installation de fractionnement d'un gaz issu de la pyrolyse d'hydrocarbures selon la revendication 5, **caractérisé en ce que** le gaz (59) issu du dernier séparateur (55) est divisé en un flux (60) réchauffé dans l'échangeur cryogénique (62) et un flux (81) détendu et refroidi dans une turbine (68) puis réchauffé dans l'échangeur cryogénique (62).

7. Installation de fractionnement d'un gaz issu de la pyrolyse d'hydrocarbures selon la revendication 5 ou 6, **caractérisée en ce que** l'échangeur cryogénique (62) est alimenté par deux flux de GNL, un flux haute pression (402) à une première pression relativement plus haute et un flux moyenne pression (401) à une seconde pression relativement plus faible.

8. Installation de fractionnement d'un gaz issu de la pyrolyse d'hydrocarbures selon la revendication 7, **caractérisé en ce que** le flux moyenne pression (401) de GNL alimentant l'échangeur cryogénique (62) est produit en dérivant une partie du flux haute pression (402) et en le détendant pour abaisser sa température, le débit du flux moyenne pression (401) dépendant de la température du flux de GNL (400) et étant d'autant plus grand que cette température est haute.

## Claims

1. A method for fractionating a gas (1) coming from the pyrolysis of hydrocarbons, containing hydrogen and hydrocarbons, particularly C₁ to C₄ hydrocarbons including ethylene, ethane and methane, possibly water and CO₂, into at least one first current (76) enriched with hydrogen and with methane and possibly at least one second current (31) made up essentially of water, including:
a) an operation (70) for compressing and drying the gases (1) through passage into a series of compressors (3, 10, 17, 24, 36), each associated with a cooler respectively (4, 11, 18, 25, 37) and a separator respectively (5, 12, 19, 26, 39), and further including a CO₂ removal step if the gas (1) contains an excessive amount thereof, in order to obtain a compressed gas (73),
b) an operation (80) for progressively cooling and partially liquefying a gas (73) obtained from operation a) for compressing and drying, by means of successive passages into a cryogenic exchanger (62) and separation in the separators (44, 46, 49, 52, 55) into:
(i) liquids respectively (71, 48, 51, 54, 56) enriched with hydrocarbons which are C₂ and higher, particularly with ethylene and with ethane, but low in hydrogen and in methane, and into:
(ii) gases respectively (45, 47, 50, 53, 59) depleted in hydrocarbons which are C₂ and higher, particularly in ethylene and in ethane, each successive passage into the cryogenic exchanger (62) bringing the gases (73, 45, 47, 50, 53) to a temperature which is relatively lower and thus enabling the more complete liquefaction of the hydrocarbons, the gas (59) coming from the last separator (55) constituting the first current (76), containing essentially hydrogen and methane and a very low quantity of ethylene and ethane, with this gas (59) then being collected, the cryogenic exchanger (62) being cooled by at least one coolant fluid (400) which is liquefied natural gas (LNG); and
c) a distillation operation (90),
**characterised in that**:
d) the gas (73) subjected to operation b) for progressive cooling and partial liquefaction is the gas coming from operation a) for compressing and drying;
e) the distillation operation c) is carried out in a column (64) supplied at different levels by the liquids (71, 48, 51, 54, 56) collected at operation b) and partially depressurised, said column (64) enabling the separation, in an upper part which is relatively cooler, of a gaseous top current (65) enriched with methane and depleted in ethylene and in ethane, and in a lower part which is relatively less cool, of a liquid bottom current (66) depleted in methane and enriched with ethylene and with ethane, the top current (65) being recycled to operation a), upstream of the compressor, the entry pressure of which is closest to the operating pressure of the column (64), the bottom current (66) being collected and constituting a third current (77), containing the largest part of the hydrocarbons which are C₂ and higher, particularly ethylene and ethane;
f) the temperature of the LNG (400) at the entry of the cryogenic exchanger (62) can vary from minus 89°C to minus 160°C; and
g) the gas (59) coming from the last separator (55), the top current (65) and the bottom current (65) are heated in the cryogenic exchanger (62).

2. A method for fractionating a gas coming from the pyrolysis of hydrocarbons according to Claim 1, **characterised in that** the gas (59) coming from the last separator (55) is divided into a flow (60) which is heated in the cryogenic exchanger (62) and a flow (61) which is depressurised and cooled in a turbine (68) then heated in the cryogenic exchanger (62).

3. A method for fractionating a gas coming from the pyrolysis of hydrocarbons according to Claim 1 or 2, **characterised in that** the cryogenic exchanger (62) is supplied by two LNG flows, a high-pressure flow (402) at a first pressure which is relatively higher and a moderate-pressure flow (401) at a second pressure which is relatively lower.

4. A method for fractionating a gas coming from the pyrolysis of hydrocarbons according to Claim 3, **characterised in that** the moderate-pressure flow (401) of LNG supplying the cryogenic exchanger (62) is produced by diverting a part of the high-pressure flow (402) and by depressurising it in order to lower its temperature, the moderate-pressure flow rate (401) being dependent on the temperature of the LNG flow (400) and being commensurately high with this temperature.

5. An installation for fractionating a gas (1) coming from the pyrolysis of hydrocarbons, containing hydrogen and hydrocarbons, particularly C₁ to C₄ hydrocarbons including ethylene, ethane and methane, possibly water and CO₂ and at least one first current (76) enriched with hydrogen and with methane, possibly at least one second current (31) made up essentially of water, including:
a) a unit (70) for compressing and drying gas (1) through passage into a series of compressors (3,10,17, 24, 36), each associated with a cooler respectively (4, 11, 18, 25, 37) and a separator respectively (5, 12, 19, 26, 39), further including a CO₂ removal step if the gas (1) contains an excessive amount thereof, in order to obtain a compressed gas (73),
b) a unit (80) for progressively cooling and partially liquefying a gas (73) obtained from the unit (70) for compressing and drying, by means of successive passages into a cryogenic exchanger (62) and separation in the separators (44, 46, 49, 52, 55) into:
(i) liquids respectively (71, 48, 51, 54, 56) enriched with hydrocarbons which are C₂ and higher, particularly with ethylene and with ethane, but low in hydrogen and in methane, and into:
(ii) gases respectively (45, 47, 50, 53, 59) depleted in hydrocarbons which are C₂ and higher, particularly in ethylene and in ethane, each successive passage into the cryogenic exchanger (62) bringing the gases (73, 45, 47, 50, 53) to a temperature which is relatively lower and thus enabling the more complete liquefaction of the hydrocarbons, the gas (59) coming from the last separator (55) constituting the first current (76), containing essentially hydrogen and methane and a very low quantity of ethylene and ethane, with this gas (59) then being collected, the cryogenic exchanger (62) being cooled by at least one coolant fluid (400) which is liquefied natural gas (LNG); and
c) a distillation unit (90),
**characterised in that**:
d) the gas (73) introduced into the unit (80) for progressive cooling and partial liquefaction is the gas coming from the unit (70) for compressing and drying;
e) said distillation unit (90) essentially includes a column (64) supplied at different levels by the liquids (71, 48, 51, 54, 56) which are collected in the unit (80) for progressive cooling and partial liquefaction and which are partially depressurised, said column (64) enabling the separation, in an upper part which is relatively cooler, of a gaseous top current (65) enriched with methane and depleted in ethylene and in ethane, and in a lower part which is relatively less cool, of a liquid bottom current (66) depleted in methane and enriched with ethylene and with ethane, the top current (65) being recycled into the compression and drying unit (70), upstream of the compressor, the entry pressure of which is closest to the operating pressure of the column (64), the bottom current (66) being collected and constituting a third current (77) containing the largest part of the hydrocarbons which are C₂ and higher, particularly ethylene and ethane;
f) the temperature of the LNG (400) at the entry of the cryogenic exchanger (62) can vary from minus 89°C to minus 160°C; and
g) the gas (59) coming from the last separator (55), the top current (65) and the bottom current (65) are heated in the cryogenic exchanger (62).

6. An installation for fractionating a gas coming from the pyrolysis of hydrocarbons according to Claim 5, **characterised in that** the gas (59) coming from the last separator (55) is divided into a flow (60) which is heated in the cryogenic exchanger (62) and a flow (61) which is depressurised and cooled in a turbine (68) then heated in the cryogenic exchanger (62).

7. An installation for fractionating a gas coming from the pyrolysis of hydrocarbons according to Claim 5 or 6, **characterised in that** the cryogenic exchanger (62) is supplied by two LNG flows, a high-pressure flow (402) at a first pressure which is relatively higher and a moderate-pressure flow (401) at a second pressure which is relatively lower.

8. An installation for fractionating a gas coming from the pyrolysis of hydrocarbons according to Claim 7, **characterised in that** the moderate-pressure flow (401) of LNG supplying the cryogenic exchanger (62) is produced by diverting a part of the high-pressure flow (402) and by depressurising it in order to lower its temperature, the moderate-pressure flow rate (401) being dependent on the temperature of the LNG flow (400) and being commensurately high with this temperature.

## Patentansprüche

1. Verfahren zum Fraktionieren eines durch die Pyrolyse von Kohlenwasserstoffen gewonnenen Gases (1), welches Wasserstoff und Kohlenwasserstoffe, insbesondere Kohlenwasserstoffe von C₁ bis C₄ einschließlich Ethylen, Ethan und Methan, möglicherweise Wasser und CO₂, aufweist, in zumindest einen ersten Strom (76), der mit Wasserstoff und mit Methan angereichert ist, möglicherweise in zumindest einen zweiten Strom (31), der im Wesentlichen aus Wasser besteht, wobei das Verfahren die folgenden Verfahrensschritte aufweist:
a) Verdichten (70) und Trocknen der Gase (1) durch Durchlaufen einer Reihe von Verdichtern (3, 10, 17, 24, 36), welche jeweils mit einer Kühleinrichtung (4, 11, 18, 25, 37) und jeweils mit einem Separator (5, 12, 19, 26, 39) verbunden sind, und wobei sie außerdem eine Stufe zur Beseitigung von CO₂ aufweisen, falls das Gas (1) davon zuviel enthält, zum Erhalt eines verdichteten Gases (73);
b) Progressives Kühlen (80) und teilweises Verflüssigen eines Gases (73), welches von dem Verfahrensschritt a) Verdichten und Trocknen erhalten wird, durch aufeinanderfolgende Durchläufe in einem Tiefsttemperatur-Wärmetauscher (62) und Separieren in Separatoren (44, 46, 49, 52, 55) in:
(i) Flüssigkeiten (71, 48, 51, 54, 56), welche jeweils mit Kohlenwasserstoffen von C₂ und höheren, insbesondere in Ethylen und in Ethan, angereichert, aber arm an Wasserstoff und Methan sind; und in:
(ii) Gase (45, 47, 50, 53, 59), welche jeweils an Kohlenwasserstoffen von C₂ und höheren, insbesondere an Ethylen und an Ethan, verarmt sind, wobei jeder aufeinanderfolgende Durchlauf in dem Tiefsttemperatur-Wärmetauscher (62) die Gase (73, 45, 47, 50, S3) auf eine relativ tiefere Temperatur hinführt und somit die Kohlenwasserstoffe voll und ganz verflüssigt, wobei das aus dem letzten Separator (55) gewonnene Gas (59) den ersten Strom (76) bildet, welcher im Wesentlichen Wasserstoff und Methan und eine sehr geringe Menge von Ethylen und Ethan aufweist, wobei dieses Gas (59) dann aufgefangen bzw. gespeichert wird, wobei der Tiefsttemperatur-Wärmerauscher (62) durch zumindest ein Kühlfluid (400), welches ein Flüssigerdgas (GNL) ist, gekühlt wird; und
c) Destillieren (90),
**dadurch gekennzeichnet, dass**:
d) das Gas (73), welches dem Verfahrensschritt b) des progressiven Kühlens und teilweisen Verflüssigens unterzogen wird, das aus dem Verfahrensschritt a) des Verdichtens und Trocknens gewonnene Gas ist.
e) der Verfahrensschritt c) des Destillierens in einer Kolonne (64), welche auf unterschiedlichen Höhen durch die bei dem Verfahrensschritt b) gespeicherten bzw. aufgefangenen und zum Teil entspannten Flüssigkeiten (71, 48, 51, 54, 56) gespeist wird, durchgeführt, wird, wobei es diese Kolonne (64) ermöglicht, in einem oben liegenden, relativ kälteren Abschnitt einen gasförmigen Kopfstrom (65), welcher mit Methan angereichert und an Ethylen und an Ethan verarmt ist, und in einem unten liegenden, relativ weniger kalten Abschnitt einen flüssigen Fußstrom (66), welcher an Methan verarmt und mit Ethylen und mit Ethan angereichert ist, zu separieren, wobei der Kopfstrom (65) im Verfahrensschritt a) in Strömungsrichtung vor dem Verdichter, dessen Eingangsdruck dem Funktionsdruck der Kolonne (64 am nächsten kommt, an diesen zurückgeführt wird, wobei der Fußstrom (66) aufgefangen bzw. gesammelt wird und einen dritten Strom (77) bildet, welcher den größten Teil von Kohlenwasserstoffen von C₂ und höher, insbesondere Ethylen und Ethan, aufweist;
f) die Temperatur des GNL (400) am Eingang des Tiefsttemperatur- Wärmetauschers (62) von minus 89°C bis minus 160°C variieren kann; und
g) das von dem letzten Separator (55) gewonnene Gas (59), der Kopfstrom (65) und der Fußstrom (66) in dem Tiefsttemperatur-Wärmetauscher (62) wiedererwärmt werden.

2. Verfahren zum Fraktionieren eines durch die Pyrolyse von Kohlenwasserstoffen gewonnenen Gases nach Anspruch 1, **dadurch gekennzeichnet, dass** das von dem letzten Separator (55) gewonnene Gas (59) in einen in dem Tiefsttemperatur-Wärmetauscher (62) wiedererwärmten Fluss (60) und in einen in einer Turbine (68) entspannten und abgekühlten, danach in dem Tiefsttemperatur-Wärmetauscher (62) wiedererwärmten Fluss (61) aufgeteilt wird.

3. Verfahren zum Fraktionieren eines durch die Pyrolyse von Kohlenwasserstoffen gewonnenen Gases nach Anspruch 1 oder 2, **dadurch gekennzeichnet, dass** der Tiefsttemperatur-Warmetauscher (62) durch zwei GNL-Strömungen, eine Hochdruckströmung (402) mit einem ersten, relativ höheren Druck und eine Mitteldruckströmung (401) mit einem zweiten, relativ geringeren Druck, gespeist wird.

4. Verfahren zum Fraktionieren eines durch die Pyrolyse von Kohlenwasserstoffen gewonnenen Gases nach Anspruch 3, **dadurch gekennzeichnet, dass** die GNL-Mitteldruckströmung (401), welche den Tiefsttemperatur-Wärmelauscher (62) speist, durch Ableiten eines Abschnitts des Hochdruckstroms (402) und Entspannen desselben zum Absenken seiner Temperatur erzeugt wird, wobei der Durchsatz der Mitteldruckströmung (401) von der Temperatur des GNL-Flusses (400) abhängig ist und umso größer ist wie diese Temperatur hoch ist.

5. Anlage zur Fraktionierung eines durch die Pyrolyse von Kohlenwasserstoffen gewonnenen Gases (1) welches Wasserstoff und Kohlenwasserstoffe, insbesondere Kohlenwasserstoffe von C₁ bis C₄ einschließlich Ethylen, Ethan und Methan, möglicherweise Wasser und CO₂, aufweist, in zumindest einen ersten Strom (76), der mit Wasserstoff und mit Methan angereichert ist, möglicherweise in zumindest einen zweiten Strom (31), der im Wesentlichen aus Wasser besteht, wobei die Anlage Folgendes aufweist:
a) eine Verdichtungs- und Trocknungseinheit (70) für das Gas (1) durch Durchlauf einer Reihe von Verdichtern (3, 10, 17, 24, 36), welche jeweils mit einer Kühleinrichtung (4, 11, 18, 25, 37) und jeweils mit einem Separator (5, 12, 19, 26, 39) verbunden sind, und wobei sie außerdem eine Stufe zur Beseitigung von CO₂ aufweisen, falls das Gas (1) davon zuviel enthält, zum Erhalt eines verdichteten Gases (73):
b) eine Einheit (80) zur progressiven Kühlung und teilweisen Verflüssigung eines von der Verdichtungs- und Trocknungseinheit (70) erhaltenen Gases (73) durch aufeinanderfolgende Durchläufe in einem Tiefsttemperatur- Wärmetauscher (62) und Separierung in Separatoren (44, 46, 49, 52, 55) in:
(j) Flüssigkeiten (71, 48, 51, 54, 56), welche jeweils mit Kohlenwasserstoffen von C₂ und höheren, insbesondere in Ethylen und in Ethan, angereichert, aber arm an Wasserstoff und Methan sind; und in:
(ii) Gase (45, 47, 50, 53, 59), welche jeweils an Kohlenwasserstoffen von C₂ und höheren, insbesondere an Ethylen und an Ethan, verarmt sind, wobei jeder aufeinanderfolgende Durchlauf in dem Tiefsttemperatur-Wärmetauscher (62) die Gase (73, 45, 47, 50, 53) auf eine relativ tiefere Temperatur hinführt und somit die Kohlenwasserstoffe voll und ganz verflüssigt, wobei das aus dem letzten Separator (55) gewonnene Gas (59) den ersten Strom (76) bildet, welcher im Wesentlichen Wasserstoff und Methan und eine sehr geringe Menge von Ethylen und Ethan aufweist, wobei dieses Gas (59) dann aufgefangen bzw. gespeichert wird, wobei der Tiefsttemperatur-Wärmetauscher (62) durch zumindest ein Kühlfluid (400), welches ein Flüssigerdgas (GNL) ist, gekühlt ist; und
c) eine Destillationseinheit (90),
**dadurch gekennzeichnet, dass**:
d) das Gas (73), welches in die Einheit (80) zur progressiven Kühlung und teilweisen Verflüssigung eingebracht ist, das aus der Verdichtungs- und Trocknungseinheit (70) gewonnene Gas ist;
e) die Destillationseinheit (90) im Wesentlichen eine Kolonne (64) aufweist, welche auf unterschiedlichen Höhen durch die in der Einheit (80) zur progressiven Kühlung und teilweisen Verflüssigung gespeicherten bzw. aufgefangenen und zum Teil entspannte Flüssigkeiten (71, 48, 51, 54, 56) gespeist wird, wobei es diese Kolonne (64) ermöglicht, in einem oben liegenden, relativ kälteren Abschnitt einen gasförmigen Kopfstrom (65), welcher mit Methan angereichert und an Ethylen und an Ethan verarmt ist, und in einem unten liegenden, relativ weniger kalten Abschnitt einen flüssigen Fußstrom (66), welcher an Methan verarmt und mit Ethylen und mit Ethan angereichert ist, zu separieren, wobei der Kopfstrom (65) in der Verdichtungs- und Trocknungseinheit (70) in Strömungsrichtung vor dem Verdichter, dessen Eingangsdruck dem Funktionsdruck der Kolonne (64) am nächsten kommt, an diesen zurückgeführt wird, wobei der Fußstrom (66) aufgefangen bzw. gesammelt wird und einen dritten Strom (77) bildet, welcher den größten Teil von Kohlenwasserstoffen von C₂ und höher, insbesondere Ethylen und Ethan, aufweist;
f) die Temperatur des GNL (400) am Eingang des Tiefsttemperatur-Wärmetauschers (62) von minus 89°C bis minus 160°C variieren kann; und
g) das von dem letzten Separator (55) gewonnene Gas (59), der Kopfstrom (65) und der Fußstrom (66) in dem Tiefsttemperatur- Wärmetauscher (62) wiedererwärmbar sind.

6. Anlage zur Fraktionierung eines durch die Pyrolyse von Kohlenwasserstoffen gewonnenen Gases nach Anspruch 5, **dadurch gekennzeichnet, dass** das von dem letzten Separator (55) gewonnene Gas (59) in einen in dem Tiefsttemperatur-Wärmetauscher (62) wiedererwärmten Fluss (60) und in einen in einer Turbine (68) entspannten und abgekühlten, danach in dem Tiefsttemperatur- Wärmetauscher (62) wiedererwärmten Fluss (61) aufgeteilt ist.

7. Anlage zur Fraktionierung eines durch die Pyrolyse von Kohlenwasserstoffen gewonnenen Gases nach Anspruch 5 oder 6, **dadurch gekennzeichnet, dass** der Tiefsttemperatur-Wärmetauscher (62) durch zwei GNL-Strömungen, eine Hochdruckströmung (402) mit einem ersten, relativ höheren Druck und eine Mitteldruckströmung (401) mit einem zweiten, relativ geringeren Druck, gespeist ist.

8. Anlage zur Fraktionierung eines durch die Pyrolyse von Kohlenwasserstoffen gewonnenen Gases nach Anspruch 7, **dadurch gekennzeichnet, dass** die GNL-Mitteldruckströmung (401), welche den Tiefsttemperatur-Wärmetauscher (62) Speist, durch Ableitung eine; AhsGhnitts des Hochdruckstroms (402) und Entspannung desselben zur Absenkung seiner Temperatur erzeugt ist, wobei der Durchsatz der Mitteldruckströmung (401) von der Temperatur des GNL-Flusses (400) abhängig ist und umso größer ist wie diese Temperatur hoch ist.
